# EUROPEAN PATENT APPLICATION

(11) **EP 4 578 452 A1**
(43) Date of publication of application: **02.07.2025**
(21) Application number: 23856975.0
(22) Date of filing: 29.06.2023
(51) Int. Cl.: A61K 31/726, A61K 9/06, A61K 9/08, A61K 31/727, A61P 27/02

(54) **CORNEAL DISORDER TREATMENT AGENT**

(30) Priority: 23.08.2022 JP 2022132848
(71) Applicant: M's Science Corporation, Kobe-shi, Hyogo 6510083 (JP)
(72) Inventor: MITA Shiro, Nishinomiya-shi, Hyogo 662-0061 (JP)
(74) Representative: Kraus & Lederer PartGmbB
(86) International application number: PCT/JP2023/024223
(87) International publication number: WO 2024/042858

(57) **Abstract**

Provided is a novel agent or composition (agent or composition for treatment and/or prevention of a corneal disorder), etc. The agent or composition contains at least one glycosaminoglycan selected from among glycosaminoglycans having an average molecular weight of 10,000 or less and analogous substance (analogs, derivatives) thereto.

## Description

### [Technical field]

The present invention relates to novel corneal disorders therapeutic agents (eye drops) and the like.

### [Background art]

The cornea is the outermost layer of an eyeball, and its surface is exposed to the outside. Therefore, the corneas are frequently injured by various causes such as foreign matter, e.g. dust, or through the application of contact lenses. Furthermore, the corneas are often injured as a result of decreased lacrimal fluid secretion, among other etiologies, with symptoms referred to as "dry eye." Corneal disorders are also frequently caused by bacterial or viral infections, allergic reactions, corneal inflammation, or diseases, such as Stevens-Johnson syndrome. Various etiologies, as summarized above, can underline corneal disorders, which include serious ones such as corneal ulcers and severe corneal epithelial cell abrasions and mild ones such as dry eye.

The basis of curing various corneal disorders, regardless of the level of severity, is that corneal epithelial cells proliferate, stretch, and migrate to cover the lesions. However, the only drugs that are practically administered to treat corneal disorders and protect the corneas are replenishing agents for the lacrimal fluid, which include artificial lacrimal fluid and moisture-retentive preparations. Therefore, drugs that are more effective against serious corneal disorders are needed. Substances that are therapeutically effective against corneal disorders have been actively explored in the past. For example, polysaccharides such as chondroitin sulfate and hyaluronic acid have been reported to be effective in treating corneal disorders (Patent documents 1, 2, and 3). The therapeutic efficacy of these polysaccharides is mainly attributable to corneal protection based on effective moisture retention (Non-patent documents 1 and 2). However, they are insufficiently effective against severe corneal disorders such as corneal ulcers. Therefore, novel therapeutic agents for corneal disorders are urgently required.

### [Prior art]

### [Patent documents]

[Patent document 1] Patent Application Publication No. H10-226704
[Patent document 2] Patent Application Publication No. 2018-8944
[Patent document 3] JP Patent No. 4942681

### [Non-patent documents]

[Non-patent document 1] Chondroitin ophthalmic solution 1% "NITTEN" package insert
[Non-patent document 2] Hyalein ophthalmic solution 0.3% package insert

### [Summary of the invention]

### [Problems to be solved by the invention]

The purpose of the present invention is to provide novel corneal disorder therapeutic agents (eye drops) and the like.

### [Means to solve the problems]

As described above, certain polysaccharides are used as eye-drops to treat corneal diseases. In particular, among such polysaccharides, those classified as glycosaminoglycans have been reported to effectively promote the healing of corneal disorders (Patent documents 1, 2, and 3).

A glycosaminoglycan is composed of repeating disaccharide units, each consisting of an amino sugar such as glucosamine and a uronic acid or galactose bonded to each other. Glycosaminoglycans in organisms vary widely in molecular weight but are generally large molecules with molecular weights ranging from several hundred thousand to several million. Several reports suggest the therapeutic efficacy of glycosaminoglycans against corneal disorders. All of them have stated that effective glycosaminoglycans must or preferably should have large molecular weights (Patent documents 1, 2, and 3). However, to the best of our knowledge, no study has demonstrated that monosaccharides or disaccharides found as basic structural units of low molecular weight (10,000 or less) glycosaminoglycans, oligosaccharides, or polysaccharides are effective for the treatment of corneal disorders. Under these circumstances, we observed that low molecular weight oligosaccharides or polysaccharides comprising component sugars of glycosaminoglycans, such as amino sugars and uronic acids, can be as effective as or more effective than glycosaminoglycans with relatively higher molecular weights for the treatment and prevention of corneal disorders, among other purposes. We, therefore, have accomplished the present invention.

More specifically, the present invention relates to the following:
[1] A therapeutic and/or prophylactic agent for corneal disorders (an agent or composition for the treatment and/or prevention of corneal disorders), wherein at least one glycosaminoglycan species is selected from glycosaminoglycans with a specific molecular weight (e.g. the average molecular weight is 10,000 or less) or a specific number of monosaccharide unit and analogous substances (analogs, derivatives, e.g. polysaccharides in which the constituent amino sugars are replaced by glucosamine and galactosamine, polysaccharides in which the constituent amino sugars are neither glucosamine or galactosamine)..
[2] The agent described in [1], wherein the glycosaminoglycan species has an average molecular weight of 8,000 or less.
[3] The agent described in [1] or [2], wherein the glycosaminoglycan species at least comprises glucosamine units if not all.
[4] The agent described in any of [1]-[3], wherein the average number of sulfate groups per monosaccharide unit constituting the glycosaminoglycan species is 0.2 or more.
[5] The agent described in any of [1]-[4], wherein the average number of sulfate groups per monosaccharide unit constituting the glycosaminoglycan species is 1.1 or more.
[6] The agent described in any of [1]-[5], wherein the glycosaminoglycan species is at least one selected from heparin (low molecular weight heparin) and heparinoid (low molecular weight heparinoid).
[7] The agent described in any of [1]-[6], wherein the glycosaminoglycan species has an average molecular weight of 8,000 or less, the average number of sulfate group per monosaccharide unit constituting the glycosaminoglycan species is 1.2 or more, and the glycosaminoglycan species is at least one selected from heparin and heparinoid.
[8] The agent described in any of [1]-[7], wherein the glycosaminoglycan species is at least one selected from dalteparin, reviparin, parnaparin, enoxaparin, danaparoid, bemiparin, nadroparin, certoparin, tinzaparin, and fondaparinux in addition to the salts of these compounds.
[9] The agent described in any of [1]-[8], wherein the glycosaminoglycan species is at least one selected from fondaparinux and fondaparinux salts.
[10] The agent described in any of [1]-[9] is in the form of ophthalmic solution, ophthalmic gel, or ophthalmic ointment.
[11] A therapeutic and/or prophylactic method (migration, extension and/or proliferation of corneal epithelial cells) using or administering (e.g. dropping onto cornea, bringing into contact with cornea) glycosaminoglycan species for corneal disorders, wherein at least one glycosaminoglycan species (or an agent or composition containing relevant glycosaminoglycan species) is selected from glycosaminoglycans with a specific molecular weight (e.g. the average molecular weight is 10,000 or less) or a specific number of monosaccharide unit and analogous substances (analogs, derivatives, e.g. polysaccharides in which the constituent amino sugars are replaced by glucosamine and galactosamine, polysaccharides in which the constituent amino sugars are neither glucosamine nor galactosamine).
[12] The method described in [11], wherein the glycosaminoglycan species has an average molecular weight of 8,000 or less.
[13] The method described in [11] or [12], wherein the glycosaminoglycan species at least comprises glucosamine units if not all.
[14] The method described in any of [11]-[13], wherein the average number of sulfate group per monosaccharide unit constituting the glycosaminoglycan species is 0.2 or more.
[15] The method described in any of [11]-[14], wherein the average number of sulfate group per monosaccharide unit constituting the glycosaminoglycan species is 1.1 or more.
[16] The method described in any of [11]-[15], wherein glycosaminoglycan species is at least one selected from heparin (low molecular weight heparin) and heparinoid (low molecular weight heparinoid).
[17] The method described in any of [11]-[16], wherein the glycosaminoglycan species has an average molecular weight of 8,000 or less, the average number of sulfate group per monosaccharide unit constituting glycosaminoglycan species is 1.2 or more, and glycosaminoglycan species is at least one selected from heparin and heparinoid.
[18] The method described in any of [11]-[17], wherein the glycosaminoglycan species is at least one selected from dalteparin, reviparin, parnaparin, enoxaparin, danaparoid, bemiparin, nadroparin, certoparin, tinzaparin and fondaparinux in addition to the salts of these compounds.
[19] The method described in any of [11]-[18], wherein the glycosaminoglycan species is at least one selected from fondaparinux and fondaparinux salts.
[20] The method described in any of [11]-[19], wherein or glycosaminoglycan species (or an agent or composition containing glycosaminoglycan species) is in the form of ophthalmic solution, ophthalmic gel, or ophthalmic ointment.
[21] At least one glycosaminoglycan species (or an agent or composition containing relevant glycosaminoglycan species) for use in the treatment and/or prevention (migration, extension and/or proliferation of corneal epithelial cells) of corneal disorders selected from glycosaminoglycans and analogs with a specific molecular weight (e.g. the average molecular weight is 10,000 or less) or a specific number of monosaccharide unit and analogous substances (analogs, derivatives, e.g. polysaccharides in which the constituent amino sugars are replaced by glucosamine and galactosamine, polysaccharides in which the constituent amino sugars are neither glucosamine or galactosamine).
[22] Glycosaminoglycan species (or an agent or composition containing such glycosaminoglycan species) described in [21], wherein the glycosaminoglycan species has an average molecular weight of 8,000 or less.
[23] Glycosaminoglycan species (or an agent or composition containing such glycosaminoglycan species) described in [21] or [22], wherein the glycosaminoglycan species at least comprises glucosamine units if not all.
[24] Glycosaminoglycan species (or an agent or composition containing such glycosaminoglycan species) described in any of [21]-[23], wherein the average number of sulfate group per monosaccharide unit constituting the glycosaminoglycan species is 0.2 or more.
[25] Glycosaminoglycan species (or an agent or composition containing such glycosaminoglycan species) described in any of [21]-[24], wherein the average number of sulfate group per monosaccharide unit constituting the glycosaminoglycan species is 1.1 or more.
[26] Glycosaminoglycan species (or an agent or composition containing such glycosaminoglycan species) described in any of [21]-[25], wherein glycosaminoglycan species is at least one selected from heparin (low molecular weight heparin) and heparinoid (low molecular weight heparinoid).
[27] Glycosaminoglycan species (or an agent or composition containing such glycosaminoglycan species) described in any of [21]-[26], wherein the glycosaminoglycan species has an average molecular weight of 8,000 or less, the average number of sulfate group per monosaccharide unit constituting glycosaminoglycan species is 1.2 or more, and glycosaminoglycan species is at least one selected from heparin and heparinoid.
[28] Glycosaminoglycan species (or an agent or composition containing such glycosaminoglycan species) described in any of [21]-[27], wherein the glycosaminoglycan species is at least one selected from dalteparin, reviparin, parnaparin, enoxaparin, danaparoid, bemiparin, nadroparin, certoparin, tinzaparin and fondaparinux in addition to the salts of these compounds.
[29] Glycosaminoglycan species (or an agent or composition containing such glycosaminoglycan species) described in any of [21]-[28], wherein the glycosaminoglycan species is at least one selected from fondaparinux and fondaparinux salts.
[30] Glycosaminoglycan species (or an agent or composition containing such glycosaminoglycan species) described in any of [21]-[29], wherein glycosaminoglycan species (or an agent or composition containing glycosaminoglycan species) is in the form of ophthalmic solution, ophthalmic gel, or ophthalmic ointment.

### [Advantageous effects of the invention]

The present invention can provide novel agents, compositions, or other similar products for treating and preventing corneal disorders.

Such an agent or composition contains a specific ingredient, e.g. a glycosaminoglycan, glycosaminoglycan-like polysaccharide, or oligosaccharide with a molecular weight of 10,000 or less as a constituent and can be effective for the treatment of corneal epithelial disorders.

Therefore, the agents, compositions, or other similar products derived from the present invention can be used preferably for treating disorders of the corneal epithelium and other purposes.

### [Description of embodiments]

### [Intended use/function]

The agents or compositions of the present invention can be used preferably for ophthalmologic and related purposes.

In particular, the agents or compositions of the present invention can be expected to ameliorate corneal disorders through the promotion of migration, extension, and/or proliferation of corneal epithelial cells.

Therefore, the agents or compositions of the present invention can be used to treat and prevent corneal disorders (such purposes may be collectively referred to as "treatment").

Corneal disorders vary widely in severity, including protracted and severe ones such as extensive corneal epithelial abrasions and subsequent development of corneal ulcers, disorders caused through the use of contact lenses or exposure to foreign bodies, and mild corneal epithelial disorders due to decreased lacrimal fluid. Causes of corneal epithelial disorders include physical damage due to surgery or foreign matter and drug-induced damage. Diseases causing corneal epithelial disorders include bacterial keratitis, herpetic keratitis, epi demic keratoconjunctivitis, dry eye, meibomian gland dysfunction, Sjogren's syndrome, Stevens-Johnson syndrome, chronic superficial keratitis, corneal erosion, superficial punctate keratopathy, filamentary keratitis, neuroparalytic keratopathy, and diabetic keratopathy.

The agents or compositions may be used for the treatment and/or prevention of one or more types of these corneal disorders.

### [Glycosaminoglycans and compositions thereof]

An agent or composition of the present invention comprises at least one chemical selected from glycosaminoglycans and analogous substances (analogs, derivatives), collectively referred to as glycosaminoglycans, glycosaminoglycan, and other species.

In general, glycosaminoglycans are polysaccharides composed of repeating disaccharide u nits, each consisting of an amino sugar and a uronic acid or galactose that are bonded to each other.

The amino sugar unit found in a glycosaminoglycan is either glucosamine or galactosamine; however, substances pertinent to the present invention (analogous glycosaminoglycans) can comprise both of these amino sugars or amino sugars in which the amino group is added to other monosaccharides such as mannose and fucose.

Amino sugars may typically comprise glucosamine units at least or may be composed of glucosamine units alone.

Uronic acids include iduronic acid and glucuronic acid.

The uronic acid component may be one or a combination of two or more uronic acids.

Among uronic acid and/or galactose units, a glycosaminoglycan species may have uronic acid units alone (e.g. with no galactose units) or in combination with other components.

Sugars constituting glycosaminoglycan species (amino sugars, uronic acids, and galactose) may be derivatized; e.g. acylated, sulfated, and cross-linked.

Illustrative embodiments of sulfation include sulfation of amino groups constituting amino sugars (N-sulfation) and sulfation of hydroxy groups of component sugars of glycosaminoglycans such as amino sugars, uronic acids, and galactose (O-sulfation).

In derivatized sugars (e.g. sulfated sugars), one amino group or hydroxy group of the same constituent sugar may be derivatized (e.g. sulfated); alternatively, two or more amino groups and/or hydroxy groups may be derivatized (e.g. sulfated).

Sulfated glycosaminoglycans may typically have sulfated amino sugar units in addition to sulfated amino sugar units, sulfated uronic acid units, and sulfated galactose units.

In a sulfated glycosaminoglycan, the level of sulfation per constituent monosaccharide, e.g. an average number of sulfate groups per monosaccharide, may be approximately 0.1 or more, preferably 0.5 or more, more preferably 1 or more, and particularly more than 1 (e.g. 1.1 or more, 1.2 or more, 1.3 or more, 1.4 or more). Note that there is no particular upper limit of the sulfation level; the level of sulfation may be 3, 2.5, 2.2, 2, 1.8, 1.7, and 1.6.

For example, approximate sulfation levels in specific glycosaminoglycan species are ~1 per monosaccharide in heparan sulfate, ~1 per monosaccharide in chondroitin sulfate, ~1.5 per monosaccharide in heparins (low molecular weight heparins), ~1.5 per monosaccharide in heparinoids (low molecular weight heparinoids), and 0 per monosaccharide in hyaluronic acid.

Glycosaminoglycans sulfated at these levels tend to serve the functions of the present invention (e.g. promotion of corneal cell migration) more efficiently, presumably in combination with molecular weights.

Sulfated glycosaminoglycans may or may not be derivatized in ways other than sulfation.

Glycosaminoglycans may be in pharmacologically acceptable forms of salts. Examples of such salts include alkali metal salts (e.g. sodium salts, and potassium salts), alkaline earth metal salts (e.g. magnesium salts, calcium salts, and barium salts), and ammonium salts.

These salts may comprise a single glycosaminoglycan salt species or a combination of two or more glycosaminoglycan salt species.

Glycosaminoglycans may be those categorized as low molecular weight polysaccharides (e.g. low molecular weight heparins and low molecular weight heparinoids) or those that include such polysaccharides.

For example, specific glycosaminoglycan species include hyaluronic acid, chondroitin sulfate, keratan sulfate, heparan sulfate, heparins, dermatan sulfate, heparinoids (heparin analogs), and salts, e.g. sodium and calcium salts.

Specific glucosamine species [products (trade names)] include dalteparin, reviparin, parnaparin, enoxaparin, danaparoid, bemiparin, nadroparin, certoparin, tinzaparin, fondaparinux, and salts, e.g. sodium and calcium salts.

As an example with a compound name, fondaparinux or its salt has the following formula [methyl O-(2-deoxy-6-O-sulfo-2-sulfoamino-α-D-glucopyranosyl)-(1→4)-O-(β-D-glucopyran osyluronic acid)-(1→4)-O-(2-deoxy-3,6-di-O-sulfo-2-sulfoamino-α-D-glucopyranosyl)-(1-4)-O -(2-O-sulfo-α-L-idopyranosyluronic acid)-(1→4)-2-deoxy-6-O-sulfo-2-sulfoamino-α-D-glucopyranoside sodium salt; molecular weight 1,728.08; and ~1.6 sulfate groups per monosaccharide].

Fondaparinux or its salt is a chemically synthesized product and can be used preferably because it is free of biological contamination.

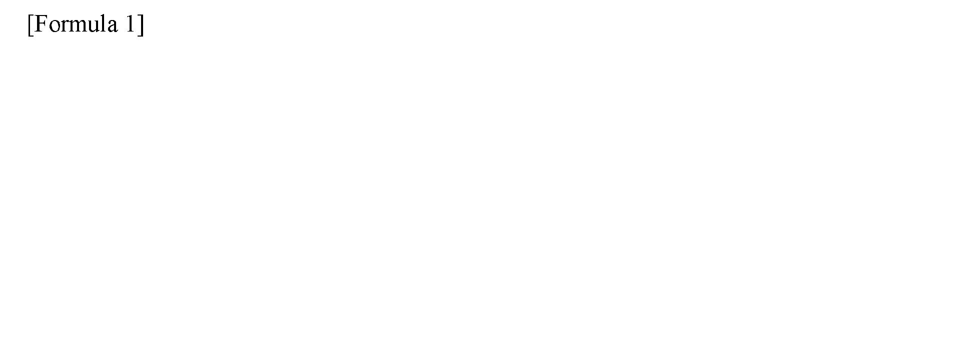

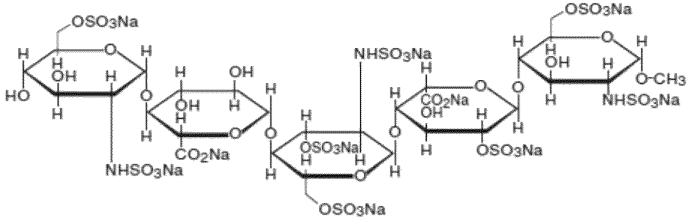

The flowing glycosaminoglycan molecular weights may be selected: 10,000 or less, 8,000 or less, or 6,000 or less in particular.

The glycosaminoglycan species may be a polysaccharide(s) (composed of more than 10 monosaccharides) or an oligosaccharide (composed of 10 or less monosaccharides).

The numbers of monosaccharides constituting a glycosaminoglycan may be e.g. 30 or less, preferably 20 or less, and even more preferably 15 or less.

The lower limit of the number of monosaccharide units of glycosaminoglycans may be 2 or more.

Glycosaminoglycans having such a relatively small molecular weight or consisting of a relatively small number of constituent sugars tend to serve the functions of the present invention (e.g. promotion of corneal cell migration) more efficiently.

The molecular weight and the number of sugars (degree of polymerization) of glycosaminoglycans may be an average value [average molecular weight, average number (average degree of polymerization)] depending on the size and the measurement method.

Various physical properties of glycosaminoglycans (e.g. average molecular weight) can be measured with conventional analytical means (e.g. high-performance liquid chromatography).

A single glycosaminoglycan species alone or a combination of two or more glycosaminoglycan species may be used.

In terms of the dosage forms that can be administered into the eye, the agents or compositions include ophthalmic solution, ophthalmic gel, or ophthalmic ointment.

The agents or compositions may be tailored (prepared) by mixing with pharmacologically acceptable additives as needed.

The ophthalmic solutions and ophthalmic gels can be formulated with commonly known methods by appropriately mixing with additives, such as tonicity agents, buffering agents, pH adjusting agents, solubilizers, thickening agents, stabilizers, and antiseptic agents as needed.

Examples of tonicity agents include glycerin, propylene glycol, sodium chloride, potassium chloride, sorbitol, and mannitol.

Examples of buffering agents include phosphoric acid or its salts (e.g. sodium monohydrogen phosphate), boric acid or its salts, citric acid or its salts (e.g. sodium citrate), tartaric acid or its salts (e.g. sodium tartrate), and ε-aminocaproic acid.

Examples of thickening and dispersing agents include cellulosic polymers such as hydro xypropyl methylcellulose or hydroxypropyl cellulose, polyvinyl alcohol, or polyvinylpyrrolidone. Examples of stabilizers include edetic acid or edetate sodium salts.

Examples of preservatives (antiseptic agents) include general-purpose sorbic acid, potassium sorbate, benzalkonium chloride, benzethonium chloride, methyl parahydroxybenzoate, propylparahydroxybenzoate, or chlorobutanol. These preservatives can also be used in combination.

The ophthalmic ointments may be ophthalmic ointment preparations commonly used in the pharmaceutical field and can be produced based on the methods described in the Ophthalmic Ointments section in the General Rules for Formulations of the Japanese Pharmacopoeia. For example, an ophthalmic ointment can be tailored (prepared) by mixing appropriate amounts of purified lanolin, white petrolatum, and liquid paraffin as ointment bases.

The pH of an ophthalmic solution may be within the range acceptable for ophthalmic preparations but is desirable to be adjusted between 4.0 and 8.5.

The agents or compositions may be administered to humans and non-human animals (e.g. mammals such as dogs and cats).

The concentrations of glycosaminoglycans in the agents or compositions are not limited and can be selected according to the appropriate dosage form, dose, and other factors. For example, the concentration of glycosaminoglycans in an agent or composition can be within the range of 0.00001%-5% (w/v).

### [Methods]

As described above, the agents or compositions (or glycosaminoglycan species) of the present invention can promote migration, stretching, in addition to proliferation of corneal epithelial cells.

Moreover, in relation to this function, the agents or compositions (or glycosaminoglycan species) of the present invention can heal corneal disorders.

### [Examples]

The following examples are described to illustrate details of the present invention; however, the scope of the present invention is not limited to these examples.

### [Experiment 1]

HCE-T (immortalized human corneal epithelial) cells [isolated and established from human corneal epithelia; obtained from RIKEN Cell Bank] were cultured in DMEM/F12 medium supplemented with 5% fetal bovine serum in a CO₂ incubator at 37°C.

HCE-T cells grown to 60%-80% confluency in a T75 flask were detached with trypsin-EDTA and collected. The collected cells were suspended in fresh DMEM/F12 medium supplemented with fetal bovine serum.

From this cell suspension, 60,000 cells per well were seeded in a 96-well multiwell-plate, in which each well was fitted with a cell seeding stopper.

After the plate was incubated overnight at 37°C in a CO ₂ incubator, the cell stoppers were removed, and the cells were washed twice with a serum-free medium. Then, the medium was replaced by a serum-free medium supplemented with the test substance or a control serum-free medium devoid of the test substance, and the cells were cultured for 28 hours.

The serum-free medium with the test substance used was a medium containing fondaparinux sodium [obtained from Selleck] at a concentration of 0.1% (w/v) or 0.3% (w/v) or a medium containing 0.5% (w/v) sodium chondroitin sulfate (obtained from Tokyo Chemical Industry; average molecular weight, ~30,000).

The cultured cells were stained with Calcein-AM (obtained from nacalai tesque), and the fluorescence emitted from HCE-T cells that migrated to the zone where the stopper was placed was measured. After the fluorescence intensity was measured, fluorescence microscopic images were acquired and analyzed using a BZ-X analyzer to measure cell migration areas.

### [Results]

The table below shows the measured fluorescence intensities and the fluorescence intensities relative to the control (relative fluorescence intensities) calculated based on the measured fluorescence intensities. A relative fluorescence intensity value is a fluorescence intensity measurement for each test substance group expressed in percentage in reference to the fluorescence intensity of the no-addition group (control) as 100%, indicating the relative level of cell migration compared to the control.

**[Table 1]**

| Condition | Fluorescence intensity | Relative fluorescence intensity (%) |
|---|---|---|
| No-addition control | 0.126 ± 0.040 | 100.00 ± 31.91 |
| Fondaparinux sodium 0.1% (w /v) | 0.183 ± 0.013 | 145.41 ± 10.19 |
| Fondaparinux sodium 0.3% (w /v) | 0.215 ± 0.004* | 170.1 ± 2.84* |
| Sodium chondroitin sulfate 0. 5% (w/v) | 0.112 ± 0.030 | 89.20 ± 23.97 |

Values shown in the table are in the form of mean and standard error of 3 measurements, and * denotes statistical significance by Dunnett's test at P < 0.05.

The results in the table above clearly show that the addition of fondaparinux sodium, which corresponds to a specific glycosaminoglycan species of the present invention, increased the fluorescence intensity and promoted cell migration. Given that the culture medium i n this experiment was not supplemented with fetal bovine serum, the increase in the fluorescence intensity is mostly attributable to stretching and migration of the cells; however, the increased cell proliferation may also have contributed to some extent.

Therefore, the fluorescence intensity of the fondaparinux sodium-added group was elevated compared to that of the no-addition control group. Fluorescence microscopic images also confirmed this finding.

The cell migration areas were calculated (measured) through analysis of images acquired with a fluorescence microscope and were used to calculate areas relative to the no-addition control group (relative migration areas).

The results are shown in the table below.

A relative migration area value is a relative cell migration area for each test substance group expressed in percentage in reference to the cell migration area of the no-addition control group as 100%.

**[Table 2]**

| Condition | Cell migration area (10⁴ µm²) | Relative cell migration area (%) |
|---|---|---|
| No-addition control | 63.6 ± 12.7 | 100.0 ± 20.0 |
| Fondaparinux sodium 0.1% (w /v) | 102.1 ± 4.2 | 160.5 ± 6.6 |
| Fondaparinux sodium 0.3% (w /v) | 107.1 ± 3.2* | 168.4 ± 5.1* |
| Sodium chondroitin sulfate 0. 5% (w/v) | 66.1 ± 1.1 | 103.9 ± 18.0 |

Values shown in the table are in the form of mean and standard error of 3 measurements, and * denotes statistical significance by Dunnett's test at P < 0.05.

The data described above (from both analysis methods) showed that fondaparinux sodium promoted migration and stretching of human corneal epithelial cells, indicating that fondaparinux sodium effectively ameliorates or heals corneal epithelial disorders.

### [Experiment 2]

As in Experiment 1, HCE-T (immortalized human corneal epithelial) cells were used to investigate the effects of test substances on the migration of HCE-T cells. The test substances used were fondaparinux sodium, high-molecular-weight sodium hyaluronate (average molecular weight 887,000), sodium heparan sulfate (average molecular weight 23,000), and low molecular weight heparin (average molecular weight 5,400). Among these test substances, fondaparinux sodium was obtained from Selleck as described above, and all other glycosaminoglycans were obtained from Iwai Chemicals Company. The concentration of each test substance was 0.3% (w/v).

Table 3 shows the results of Calcein-AM staining of the viable cells that migrated to the zone inside the stopper.

**[Table 3]**

| Condition | Fluorescence intensity | Relative fluorescence intensity (%) |
|---|---|---|
| No-addition control | 0.122 ± 0.034 | 100.00 ± 19.48 |
| Fondaparinux sodium | 0.290 ± 0.028* | 237.36 ± 22.53* |
| High-molecular-weight sodium hyaluronate | 0.176 ± 0.028 | 143.67 ± 22.72 |
| Sodium heparan sulfate | 0.218 ± 0.026 | 178.72 ± 21.02 |
| Low molecular weight heparin | 0.359 ± 0.083 | 293.94 ± 68.25 |

Table 4 shows the areas of cells that migrated into the stopper, calculated from images as in Experiment 1.

**[Table 4]**

| Condition | Cell migration area (10⁴ µm²) | Relative cell migration area (%) |
|---|---|---|
| No-addition control | 38.9±4.0 | 100.0±10.2 |
| Fondaparinux sodium | 65.9±6.4 | 169.4±16.4 |
| High-molecular-weight sodium hyaluronate | 50.9±17.4 | 130.9±4.5 |
| Sodium heparan sulfate | 52.8±2.6 | 135.9±6.7 |
| Low molecular weight heparin | 78.4±12.3* | 201.6±31.6* |

Values shown in the table are in the form of mean and standard error of 4 measurements, and * denotes statistical significance by Dunnett's test at P < 0.05.

The above results indicate that low molecular weight heparin, as well as fondaparinux, have a strong stimulatory effect on the migration of human corneal epithelial cells. This finding indicates that, in general, glycosaminoglycans (particularly sulfated ones) with small molecular weights (molecular weights of 10,000 or less) potently promote the migration of human corneal epithelial cells. Although a polysaccharide with a larger molecular weight has been considered to have a stronger promoting effect on the migration and stretching of corneal epithelial cells, these data revealed that polysaccharides with small molecular weights (e.g. 10,000 or less) had strong effects on corneal epithelial cells.

### [Industrial applicability]

The present invention can provide novel agents, compositions, or the like for treating corneal disorders.

## Claims

1. A therapeutic and/or prophylactic agent for corneal disorders comprising at least one glycosaminoglycan species selected from glycosaminoglycans with average molecular weights of 10,000 or less and analogous substances.

2. The agent of Claim 1, wherein the glycosaminoglycan species has an average molecular weight of 8,000 or less.

3. The agent of Claim 1 or 2, wherein the glycosaminoglycan species comprises glucosamine units at least.

4. The agent of Claim 1 or 2, wherein the average number of sulfate groups per monosaccharide unit constituting the glycosaminoglycan species is 0.2 or more.

5. The agent of Claim 1 or 2, wherein the average number of sulfate groups per monosaccharide unit constituting the glycosaminoglycan species is 1.1 or more.

6. The agent of Claim 1 or 2, wherein the glycosaminoglycan species is at least one selected from heparins and heparinoids.

7. The agent of Claim 1 or 2, wherein the glycosaminoglycan species has an average molecular weight of 8,000 or less, the average number of sulfate groups per monosaccharide unit constituting the glycosaminoglycan species is 1.2 or more, and the glycosaminoglycan species is at least one selected from heparins and heparinoids.

8. The agent of Claim 1 or 2, wherein the glycosaminoglycan species is at least one selected from dalteparin, reviparin, parnaparin, enoxaparin, danaparoid, bemiparin, nadroparin, certoparin, tinzaparin, fondaparinux, and salts of these compounds.

9. The agent of Claim 1 or 2, wherein the glycosaminoglycan species is at least one selected from fondaparinux and fondaparinux salts.

10. The agent of Claim 1 or 2, which is in the form of ophthalmic solution, ophthalmic gel, or ophthalmic ointment.
